# EUROPÄISCHE PATENTANMELDUNG

(11) **EP 2 522 346 A1**
(43) Veröffentlichungstag der Anmeldung: **14.11.2012**
(21) Anmeldenummer: 12003455.8
(22) Anmeldetag: 04.05.2012
(51) Int. Cl.: A61K 31/353, A61K 36/84, A61K 36/752, A61K 36/185, A61P 25/20

(54) **Wirkstoffkombinationen von Hesperidin zur Behandlung von Schlafstörungen**

(30) Priorität: 07.05.2011 DE 102011100832
(71) Anmelder: Dr. Loges + Co. GmbH, 21423 Winsen, Luhe (DE)
(72) Erfinder: Biller, Andreas, 21435 Stelle/ Ashausen (DE); Dimpfel, Wilfried, 35578 Wetzlar (DE)
(74) Vertreter: Henrion, Oliver

(57) **Zusammenfassung**

Die vorliegende Erfindung betrifft Wirkstoffkombinationen, enthaltend Hesperidin, dessen pharmazeutisch annehmbare Salze und/oder dessen Aglykon Hesperetin und mindestens einen weiteren Wirkstoff zur Behandlung von Schlafstörungen, sowie Schlafmittel oder Nahrungsergänzungsmittel umfassend eine Wirkstoffkombination, enthaltend Hesperidin, dessen pharmazeutisch annehmbare Salze und/oder dessen Aglykon Hesperetin und mindestens einen weiteren Wirkstoff zur Behandlung von Schlafstörungen und die Verwendung von Wirkstoffkombinationen, enthaltend Hesperidin, dessen pharmazeutisch annehmbare Salze und/oder dessen Aglykon Hesperetin und mindestens einen weiteren Wirkstoff zur Behandlung von Schlafstörungen.

## Beschreibung

Die vorliegende Erfindung betrifft Wirkstoffkombinationen, enthaltend Hesperidin, dessen pharmazeutisch annehmbare Salze und/oder dessen Aglykon Hesperetin und mindestens einen weiteren Wirkstoff zur Behandlung von Schlafstörungen und die Verwendung von Wirkstoffkombinationen, enthaltend Hesperidin, dessen pharmazeutisch annehmbare Salze und/oder dessen Aglykon Hesperetin und mindestens einen weiteren Wirkstoff zur Behandlung von Schlafstörungen. Die Erfindung betrifft auch ein Schlafmittel oder ein Nahrungsergänzungsmittel enthaltend eine Wirkstoffkombination, enthaltend Hesperidin, dessen pharmazeutisch annehmbare Salze und/oder dessen Aglykon Hesperetin und mindestens einen weiteren Wirkstoff. Vorzugsweise enthalten die Wirkstoffkombinationen Hesperidin in Form von Pflanzenextrakten, wie Zitrusfruchtextrakten, insbesondere Orangenextrakt aber auch Extrakten von grünem Blattgemüse, wie Spinat oder Grünkohl, und deren verschiedenen Herstellungsformen. Besonders bevorzugt sind Kombinationen von Hesperidin dessen pharmazeutisch annehmbare Salze und/oder dessen Aglykon Hesperetin, ggf. als Orangenextrakt, mit Extrakten der Baldrianwurzel und/oder Hopfenextrakt.

Hesperidin ist ein Glycosid aus den beiden Flavonen Hesperetin und Disaccharid Rutinose und zählt zur Gruppe der Flavonoide, insbesondere der Bioflavonoide. Der IUAPC-Name von Hesperidin ist (2S)-7-((6-O-(6-Desoxy-α-L-mannopyranosyl)- β-D-glucopyranosyl)oxy)- 2,3-dihydro-5-hydroxy- 2-(3-hydroxy-4-methoxyphenyl)- 4H-1-benzopyran-4-on (CAS-Nummer 520-26-3) entsprechend der Summenformel C₂₈H₃₄O₁₅ und der allgemeinen Strukturformel:

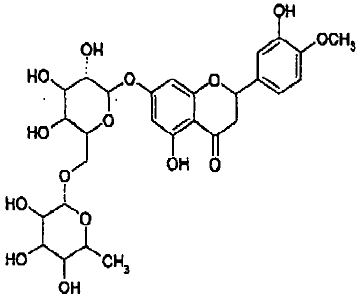

Hesperidin ist vor allem in Zitrusfrüchten, wie Orangen, Zitronen, Grapefruits und Mandarinen, enthalten. Die Schalen und membranösen Anteile der Zitrusfrüchte' haben dabei die höchste Konzentration an Hesperidin. Das Aglykon Hesperetin entsteht im Darm durch die Tätigkeit von Bakterien.

Hesperidin oder Hesperetin können erfindungsgemäß auch In Form pharmazeutisch annehmbarer Salze oder Komplexe eingesetzt werden. Die Herstellung solcher Salze erfolgt in an sich bekannter Weise. Als Salzbildner kommen alle üblichen pharmazeutisch annehmbaren Säuren bzw. Anionen in Frage. Insbesondere können auch Chalkone verwendet werden. Auch eine Kopplung an Glucuronsäure kann verwendet werden: Insbesondere kann auch Hesperidin (Glycosid) ohne seine Zuckerkomponente als Hesperetin (Aglycon) verwendet werden. Umfaßt sind ebenfalls MetabolIte und Prodrugs von Hesperidin oder Hesperetin.

Bekannt sind antioxidative, antiinflammatorische, antimikrobielle, antivirale und gefäßschützende Eigenschaften von Hesperidin (Garg A, Garg S, Zaneveld LJD, Singla AK (2001) Chemistry and Pharmacology of the Citrus Bioflavonoid Hesperidin. Phytother. Res. 15: 655-669) sowie die Verwendung von Hesperidin zur Behandlung von Epilepsie (EP 1721 613 B1).

Hesperidin enthaltende Pflanzenextrakte, wie Zitrusfruchtextrakte, insbesondere Orangenextrakt werden zu verschiedenen Zwecken hergestellt, vor allem im Bereich von Nahrungsergänzungsmitteln, um eventuell vorhandene wirksame Inhaltsstoffe in hoch konzentrierter Form anzubieten.

Die derzeit üblichen Arzneimittel zur Behandlung von Schlafstörungen haben ein breites Nebenwirkungsspektrum. Es besteht daher ein großer Bedarf nach verbesserten Behandlungsmöglichkeiten, sei es als Nahrungsergänzungsmittel oder sei es als Arzneimittel mit einer guten Wirksamkeit bei möglichst geringer Nebenwirkungsrate. Diese geringe Nebenwirkungsrate ist bei Verabreichung eines Naturstoffs oder vorzugsweise einer Kombination von Naturstoffen eher zu erwarten.

Überraschend wurde nunmehr gefunden, daß Hesperidin bzw. Hesperidin enthaltende Pflanzenextrakte eine biologische oder pharmakologische Wirkung in Bezug auf die Behandlung von Schlafstörungen in Kombination mit mindestens einem weiteren Wirkstoff aufweisen. Es hat sich gezeigt, daß Hesperidin (in Form von Orangenextrakt) in der Lage ist, bei geringer oder fehlender Eigenwirkung, die Wirkung von Schlafmitteln, insbesondere pflanzlichen Schlafmitteln zu verstärken bzw. zu potenzieren. Erst die Anwendung der völlig "bias" - freien Untersuchung im Tele-Stereo-EEG der Ratte erbrachte im Rahmen von Versuchen mit anderer Zielsetzung den Hinweis auf das Vorhandensein einer derartigen potenzierenden Wirkung. Die überraschend gefundene Wirkung wurde in vivo an der Ratte dokumentiert und in der Folge an Menschen mit Schlafstörungen verifiziert.

Die Erfindung betrifft daher Wirkstoffkombinationen, enthaltend Hesperidin, dessen pharmazeutisch annehmbare Salze und/oder dessen Aglykon Hesperetin und mindestens einen weiteren Wirkstoff zur Behandlung von Schlafstörungen. Bevorzugt sind Wirkstoffkombinationen zur oralen Anwendung. Die Erfindung betrifft auch die Wirkstoffkombinationen zur Verwendung bei der Behandlung von Schlafstörungen.

Im Rahmen der vorliegenden Erfindung sollen, sofern nicht anders angegeben, von dem Begriff "Hesperidin" alle vorgenannten Erscheinungsformen umfaßt sein. Besonders bevorzugt wird Hesperidin in Form von Hesperidin enthaltenden Pflanzenextrakten eingesetzt. Beispielhaft genannt seien Zitrusfruchtextrakte, insbesondere Orangenextrakt, aber auch Extrakte von grünem Blattgemüse, wie Spinat oder Grünkohl oder von Olivenblättern. Eingesetzt werden verschiedene Herstellungsformen der Extrakte, wie feste, flüssige, wässrige, ölige und/oder alkoholische Extrakte. Übliche Gehalte von Hesperidin in Extrakten liegen von nicht unter 30 Gew.%, bevorzugt nicht unter 50 Gew.-%, insbesondere nicht unter 60 Gew.-% und bis über 90 Gew.%. Bevorzugt werden hochkonzentrierte oder hochgereinigte Extrakte eingesetzt, die sich durch einen hohen Gehalt an Hesperidin auszeichnen. Derartige Extrakte können auf an sich bekannte Weise hergestellt werden. Unter hohem Gehalt an Hesperidin sollen vorliegend Gehalte größer gleich 70%, bevorzugt größer gleich 90%, insbesondere bevorzugt größer gleich 95 Gew.%, beispielsweise 96 Gew.-%, 97 Gew.% und 98 Gew.-% verstanden werden.

Der mindestens eine weitere Wirkstoff ist dabei ein Wirkstoff, der zur Behandlung von Schlafstörungen eingesetzt wird. Der Begriff umfaßt vorliegend natürliche Wirkstoffe, insbesondere pflanzliche Wirkstoffe, sowie Wirkstoffgemische. Beispielhaft genannt seien Baldrian(wurzel), Hopfen(zapfen), Johanniskraut, Melisse, Passionsblume, Kava kava, Lavendel, kalifornischer Mohn, Schwarznessel, sowie Kombinationen davon, bevorzugt pflanzliche Wirkstoffe, insbesondere von Baldrian(wurzel) und/oder Hopfen(zapfen). Die Wirkstoffe können in beliebiger Form eingesetzt werden. Bevorzugt werden die pflanzlichen Wirkstoffe in Form von Pflanzenextrakten, wie festen, flüssigen, wässrigen, öligen, alkoholischen Extrakten eingesetzt. Die Extrakte werden auf dem Fachmann geläufige Art und Weise hergestellt, wie z.B. wässrige Extraktion oder alkoholische Extraktion.

Unter Schlafstörungen sollen im Rahmen der vorliegenden Erfindung insbesondere Schwierigkeiten beim Einschlafen, Störungen des Durchschlafens und vorzeitiges Erwachen verstanden werden.

Die erfindungsgemäßen Wirkstoffkombinationen enthalten Hesperidin und mindestens einen weiteren Wirkstoff in einem wirksamen Verhältnis, bevorzugt in einem therapeutisch wirksamen Verhältnis, insbesondere in einem synergistisch wirksamen Verhältnis. Bevorzugt enthalten die erfindungsgemäßen Wirkstoffkombinationen ca.10 - 60 Gew.-%, bevorzugt ca. 15-50 Gew.-%, insbesondere ca. 20 - 40 Gew.-% Hesperidin in den oben genannten Erscheinungsformen bezogen auf das Gesamtgewicht der Wirkstoffkombination, bevorzugt gerechnet als Hesperidin in Reinform.

Besonders bevorzugt sind Wirkstoffkombinationen enthaltend Hesperidin in Form eines Zitrusfruchtextraktes, insbesondere Orangenextrakt und den einen weiteren Wirkstoff in Form eines Extrakts von Baldrian(wurzel) und/oder Hopfen(zapfen).

Die vorliegende Erfindung betrifft auch die Verwendung von Wirkstoffkombinationen, enthaltend Hesperidin, dessen pharmazeutisch annehmbare Salze und/oder dessen Aglykon Hesperetin und mindestens einen weiteren Wirkstoff zur Behandlung von Schlafstörungen. Die Erfindung betrifft auch die Verwendung erfindungsgemäßer Wirkstoffkombinationen als Schlafmittel. In diesem Zusammenhang ist auch die Herrichtung der erfindungsgemäßen Wirkstoffkombinationen als Schlafmittel umfaßt. Die Erfindung betrifft auch die Verwendung von Hesperidin, dessen pharmazeutisch annehmbare Salze und/oder dessen Aglykon Hesperetin als Wirkungsverstärker (Synergist) für Wirkstoffe zur Behandlung von Schlafstörungen im Sinne der vorliegenden Erfindung, vorzugsweise entsprechender pflanzlicher Wirkstoffe, wie insbesondere von Baldrian(wurzel) und/oder Hopfen(zapfen), besonders bevorzugt ist die Verwendung von Orangenextrakt als Wirkungsverstärker für Baldrian(wurzel)- und/oder Hopfen(zapfen)-Extrakt In Bezug auf die Behandlung von Schlafstörungen.

Weiterer Gegenstand der vorliegenden Erfindung sind Schlafmittel oder Nahrungsergänzungsmittel enthaltend eine Wrkstoflkombination, enthaltend Hesperidin, dessen pharmazeutisch annehmbare Salze und/oder dessen Aglykon Hesperetin und mindestens einen weiteren Wirkstoff zur Behandlung von Schlafstörungen.

Zusätzlicher Gegenstand der Erfindung ist ein Verfahren zur Behandlung von Schlafstörungen umfassend die Gabe einer erfindungsgemäßen Wirkstoffkombination oder eines erfindungsgemäßen Schlafmittels an einen Patienten; der eine solche Behandlung benötigt in einer therapeutisch wirksamen Menge.

Die Erfindung bezieht sich auch auf sämtliche Kombinationen von bevorzugten Ausgestaltungen, soweit diese sich nicht gegenseitig ausschließen. Die Angaben "etwa" oder "ca." in Verbindung mit einer Zahlenangabe bedeuten, daß zumindest um 10 % höhere oder niedrigere Werte oder um 5 % höhere oder niedrigere Werte und in jedem Fall um 1 % höhere oder niedrigere Werte eingeschlossen sind. Soweit nichts anderes angegeben ist oder sich aus dem Zusammenhang zwingend anders ergibt, beziehen sich Prozentangaben auf das Gewicht, im Zweifel auf das Gesamtgewicht der Mischung.

Die erfindungsgemäßen Wirkstoffkombinationen. Nahrungsergänzungsmittel bzw. Schlafmittel werden bevorzugt oral verabreicht. Es ist jedoch auch eine Verabreichung auf anderem Wege, z.B. peroral, topisch, parenteral, intravenös, intramuskulär, subkutan, nasal, inhalativ, rektal oder transdermal möglich.

Zur Verabreichung kann das erfindungsgemäße Schlafmittel z.B. in Form von Tabletten, Kapseln, Pillen, Dragees, Granulaten, Suppositorien, Pellets, Lösungen, Dispersionen formuliert werden, wobei optional pharmazeutisch annehmbaren. Hilfs- und Trägerstoffen enthalten sein können.

Liegt das erfindungsgemäße Schlafmittel in oraler Form vor, so enthält es besonders bevorzugt eine Kombination der Extrakte im Verhältnis Baldrianextakt : Hopfenextrakt : Orangenextrakt wie ca. 40 : 20 : 40. Andere Mischungsverhältnisse sind möglich und können vom Fachmann mittels einfacher Versuche ermittelt werden.

Die erfindungsgemäßen Schlafmittel, die Hesperidin, insbesondere in Form von Orangenextrakt als potenzierende Komponente enthalten, werden normalerweise gemäß herkömmlichen Methoden hergestellt und in einer pharmazeutisch geeigneten Form verabreicht.

Zum Beispiel können die festen oralen Formen zusammen mit dem Wirkstoff Streckstoffe, z.B. Lactose, Dextrose, Saccharose, Cellulose, Maisstärke oder Kartoffelstärke; Gleitmittel, z.B. Silikat, Talk, Stearinsäure, Magnesium- oder Calciumstearat und/oder Polyethylenglykole; Bindemittel, z.B. Stärken, Gummi arabicum, Gelatine, Methylcellulose, Carboxymethylcellulose oder Polyvinyipyrrolidon: Quell- oder Sprengmittel, z.B. Stärke, Alginsäure, Alginate oder Natriumstärkeglykolate, aufschäumende Mischungen; Farbstoffe; Soßungsmittel; Benetzungsmittel, wie Lecithin, Polysorbate, Laurylsulfate, und im allgemeinen nicht-toxische und pharmakologisch inaktive Substanzen, die in pharmazeutischen Formulierungen verwendet werden, enthalten.

Die pharmazeutischen Zubereitungen können in bekannter Weise hergestellt werden, z.B. mittels Mischen, Granulieren, Tablettieren, Zuckerbeschichtungs- oder Überzugsbeschichtungsverfahren.

Die flüssigen Dispersionen zur oralen Verabreichung können z.B. Sirupe, Emulsionen und Suspensionen sein.

Der Sirup kann als Träger z.B. Saccharose oder Saccharose mit Glycerin und/oder Mannitol und/oder Sorbitol enthalten.

Die Suspensionen und die Emulsionen können als Träger z.B. ein natürliches Harz, Agar, Natriumalginat, Pectin, Methylcellulose, Carboxymethylcellulose oder Polyvinylalkohol enthalten.

Die Suspensionen oder Lösungen für intramuskuläre Injektionen können zusammen mit dem Wirkstoff einen pharmazeutisch annehmbaren Träger, z.B. steriles Wasser, Olivenöl, Ehyloleat, Glykofe, z.B. Propylenglykol, und, falls gewünscht, eine geelgnete Menge an Lidocain-Hydrorhlorid enthalten.

Die Lösungen zur intravenösen Injektion oder Infusion können als Träger z.B. steriles Wasser enthalten oder sie können bevorzugt in Form von sterilen, wässrigen, isotonischen Salzlösungen vorliegen.

Die Suppositorien können zusammen mit dem Wirkstoff einen pharmazeutisch annehmbaren Träger, z.B. Kakaobutter, Polyethylenglykol, einen Polyoxyethylensorbitol-Fettsäureester oder Lecithin enthalten.

Zusammensetzungen für die topische Applikation, z.B. Cremes, Lotionen oder Pasten, können durch Mischen des Wirkstoffs mit einem herkömmlichen ölhaltigen oder emulgierenden Träger hergestellt werden.

Die Dosierungseinheit des Arzneimittels kann beispielsweise enthalten:
bei peroralen Arzneiformen:
   bevorzugt 200 bis 800 mg Baldrianwurzelextrakt, besonders bevorzugt 400 bis 800 mg, insbesondere 400 bis 600 mg, speziell 500 bis 600 mg, plus 200 bis 800 mg, besonders bevorzugt 300 bis 600 mg, insbesondere 400 bis 600 mg Orangenextrakt sowie 50 bis 300 mg, besonders bevorzugt 100 bis 200 mg Hopfenextrakt pro Tagesdosis.
   Die Tagesdosis kann beispielsweise in 1 bis 3 Einzeldosen, vorzugsweise In zwei Einzeldosen, täglich verabreicht werden.
bei parenteralen Arzneiformen (zum Beispiel intravenös, subkutan, intramuskulär):
   bevorzugt 200 bis 800 mg, besonders bevorzugt 400 - 800 mg, insbesondere 600 mg Baldrianwurzelextrakt, plus 200 mg Orangenextrakt sowie 100 mg Hopfenextrakt pro Tagesdosis.
   Die Tagesdosis kann beispielsweise in 1 bis 3 Einzeldosen, vorzugsweise in einer Einzeldosis, täglich verabreicht werden.
bei Arzneiformen zur rektalen Applikation:
   bevorzugt 200 bis 800 mg Baldrianwurzelextrakt, besonders bevorzugt 400 bis 800 mg, insbesondere 400 bis 600 mg, speziell 500 bis 600 mg, plus 200 bis 800 mg, besonders bevorzugt 300 bis 600 mg, insbesondere 400 bis 600 mg Orangenextrakt, sowie 50 bis 300 mg, besonders bevorzugt 100 bis 200 mg Hopfenextrakt pro Tagesdosis.
   Die Tagesdosis kann beispielsweise in 1 bis 3 Einzeldosen, vorzugsweise in zwei Einzeldosen, täglich verabreicht werden.
bei Arzneiformen zur Applikation auf die Haut und Schleimhäute (z.B, Lösungen, Lotlonen, Emulsionen, Salben usw.):
   bevorzugt 200 bis 800 mg Baidrianwurzoiextrakt, besonders bevorzugt 400 bis 800 mg, insbesondere 400 bis 600 mg, speziell 500 bis 600 mg, plus 200 bis 800 mg, besonders bevorzugt 300 bis 600 mg, insbesondere 400 bis 600 mg Orangenextrakt sowie 50 bis 300 mg, besonders bevorzugt 100 bis 200 mg Hopfenextrakt pro Tagesdosis.
   Die Tagesdosis kann beispielsweise in 1 bis 3 Einzeldosen, vorzugsweise in zwei Einzeldosen, täglich verabreicht werden.
als Nahrungsergänzungsmittel:
   bevorzugt 200 bis 800 mg Baldrianwurzelextrakt, besonders bevorzugt 400 bis 600 mg, insbesondere 500 bis 600 mg, plus 200 bis 800 mg, besonders bevorzugt 300 bis 600 mg, insbesondere 400 bis 600 mg Orangenextrakt sowie 50 bis 300 mg, besonders bevorzugt 100 bis 200 mg Hopfenextrakt pro Tagesdosis.
   Die Tagesdosis kann beispielsweise in 1 bis 3 Einzeldosen, vorzugsweise in zwei Einzeldosen, täglich als Zugabe zu Getränken verabreicht werden.

Bei Verwendung weiterer Komponenten, z.B. Extrakte, muß die verwendete Menge in einer dem Fachmann bekannten Weise entsprechend angepaßt werden.

Nachfolgend wird eine kurze Beschreibung der Abbildungen gegeben.

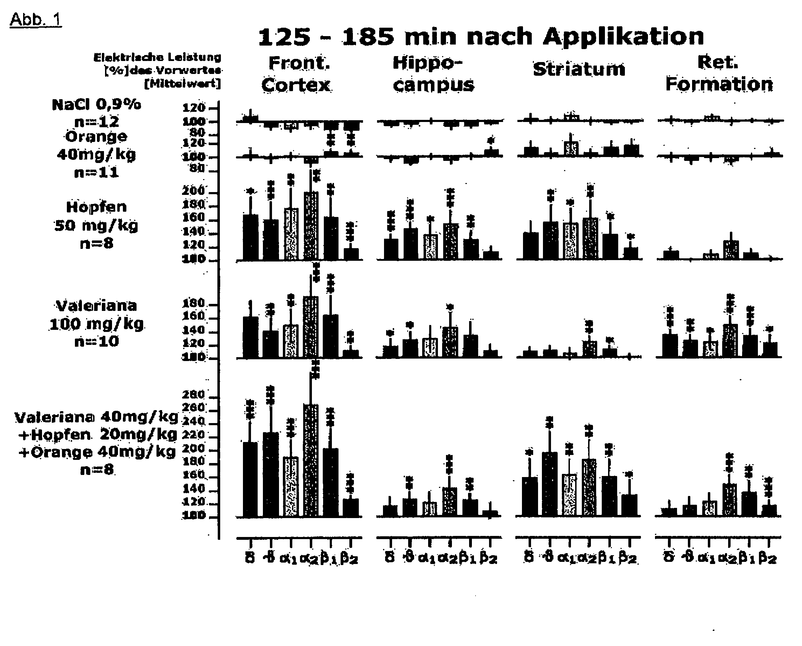

Abb. 1 zeigt Änderungen der spektralen Leistungsdichte in vier Hirnregionen der Ratte nach Verabreichung von Kochsalz, Orangenextrakt, Hopfenextrakt, Baldrianextrakt (Valeriana) sowie einer Kombination dieser drei Pflanzenextrakte. Die y-Achse zeigt die Leistungsdichte in % der Änderung vom 45-minütigen Vorwert vor Verabreichung der Extrakte. Die x-Achse gibt die Frequenzbereiche nach der Fast Fourier Transformation der Daten an: von links nach rechts: delta, theta, alpha1, alpha2, beta1 und beta2. Mittelwerte von n=x Tieren. Statistische Signifikanzen: * entspricht einer Irrtumswahrscheinlichkeit von p<0.1; ** entsprechen p<0.05; *** entsprechen p<0.01. Je höher die Leistungsdichte, umso tiefer ist der Schlaf_

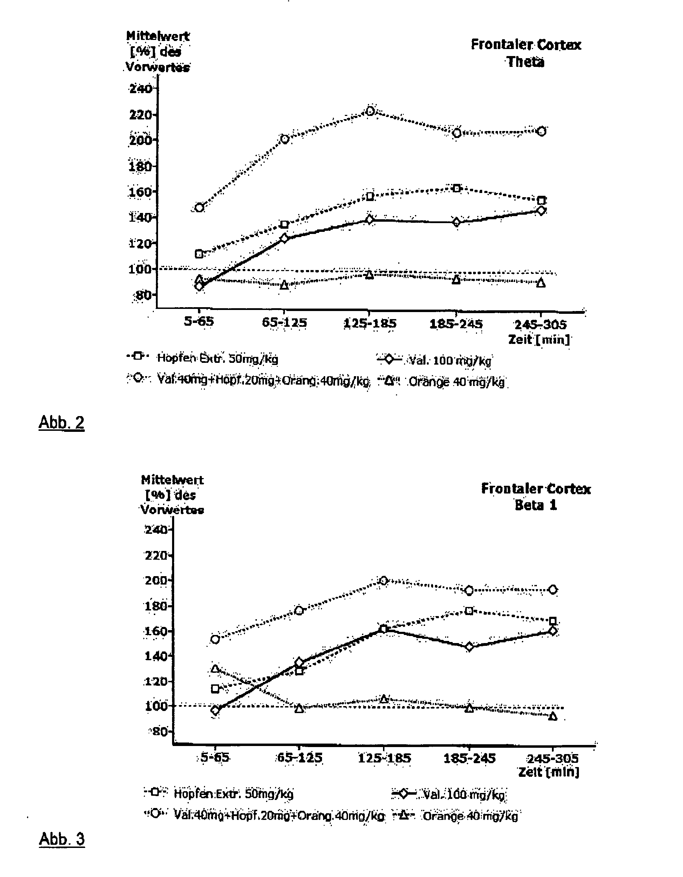

Abb. 2 zeigt den zeitlichen Verlauf der theta Wellen über 5 Stunden nach Verabreichung der einzelnen Extrakte in höherer Dosierung im Vergleich mit der Dreierkombination von Baldrian-, Hopfen- und Orangenextrakt bei der Ratte. Die y-Achse zeigt die Leistungsdichte in % der Änderung vom 45-minütigen Vorwert vor Verabreichung der Extrakte. Die Ergebnisse zeigen, daß Orangenextrakt keine Eigenwirkung auf diese Frequenz hat, jedoch in Kombination mit den anderen beiden Extrakten bei gleicher Dosierung eine maximale Wirkung erreicht wird.

Abb. 3 zeigt den zeitlichen Verlauf der beta1 Wellen über 5 Stunden nach Verabreichung der einzelnen Extrakte in höherer Dosierung im Vergleich mit der Dreierkombination von Baldrian-, Hopfen- und Orangenextrakt bei der Ratte. Die y-Achse zeigt die Leistungsdichte in % Änderung vom 45 minütigen Vorwert vor Verabreichung der Extrakte. Orangenextrakt zeigt keine Eigenwirkung auf diese Frequenz, erreicht jedoch in Kombination mit den anderen beiden bei gleicher Dosierung eine maximale Wirkung (Val=Baldrianwurzelextrakt).

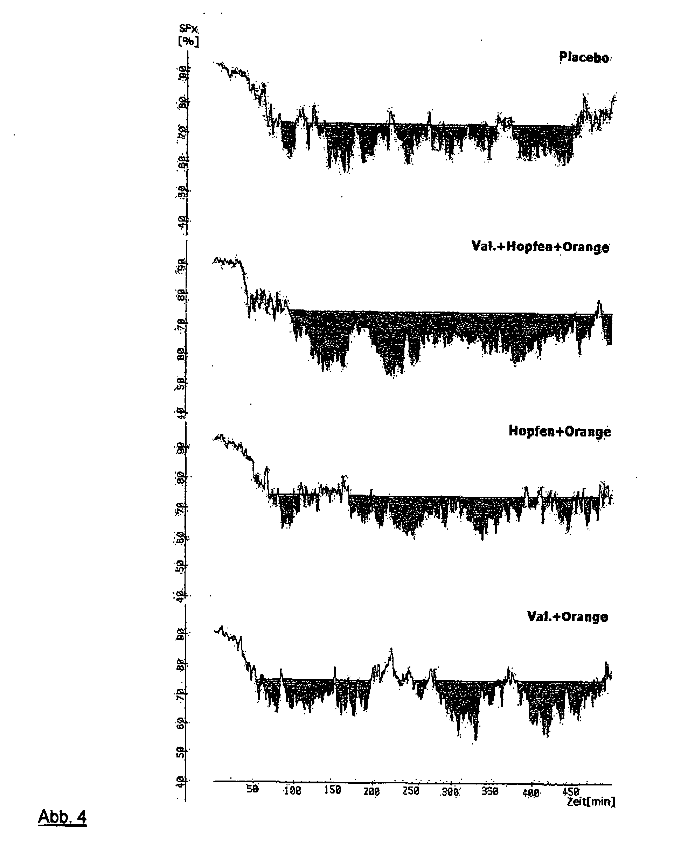

Abb. 4 zeigt den zeitlichen Verlauf des mittleren spektralen Frequenzindexes "SFx" (auf Grundlage der theta und beta Wellen berechnet) über die ganze Nacht bei n=12 Patienten (Val+Orange: n=11) mit Schlafstörungen in Gegenwart einer Kombination von Hopfen- und Orangenextrakt, Baldrian- und Orangenextrakt sowie einer Kombination aller drei Extrakte. Je niedriger der SFx Wert auf der Ordinate, umso tiefer ist der Schlaf. Die Gegenwart von Orangenextrakt verbessert den Schlaf sowohl in Kombination mit Baldrian wie auch mit Hopfen. Die beste Wirkung zeigt die Dreierkombination, wie auch schon an der Ratte dokumentiert.

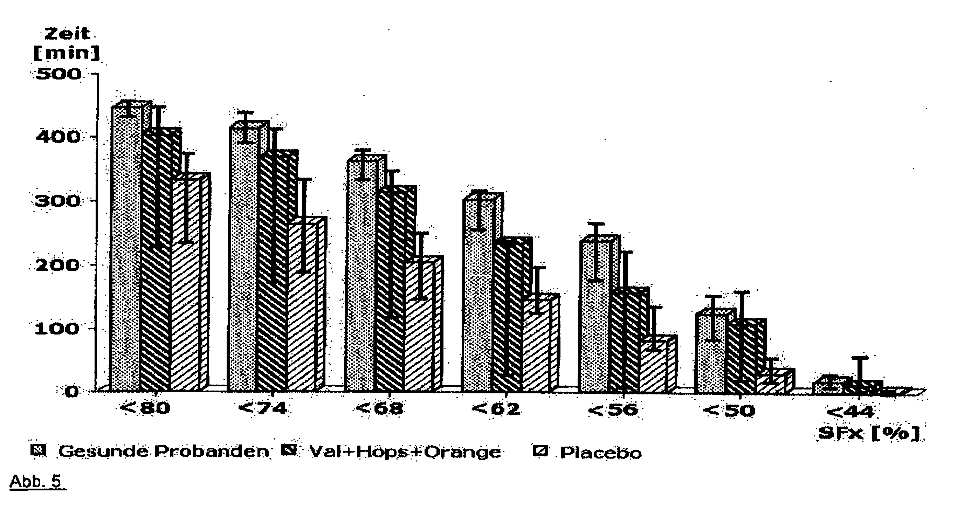

Abb. 5 zeigt eine Analyse der Zeit, die ein Patient bei einer definierten Schlaftiefe verbringt. Im Vergleich zu Placebo (vordere Balkenreihe) bewirkt die Dreierkombination von Baidrijnn-, Hopfen- und Orangenextrakt (mittlere Balkenreihe), daß unterhalb aller definierten Schlaftiefen von SFx Werten von <80% bis <44 % längere Zeit verbracht wird (s. Ordinate). Die hintere Balkenreihe zeigt Vergleichswerte von gesunden Probanden ohne Schlafstörungen. Die Werte der Dreierkombination haben sich dabei deutlich in Richtung der Werte gesunder Probanden verschoben. Auch die Streuung der Daten nimmt im Vergleich zu Placebo ab.

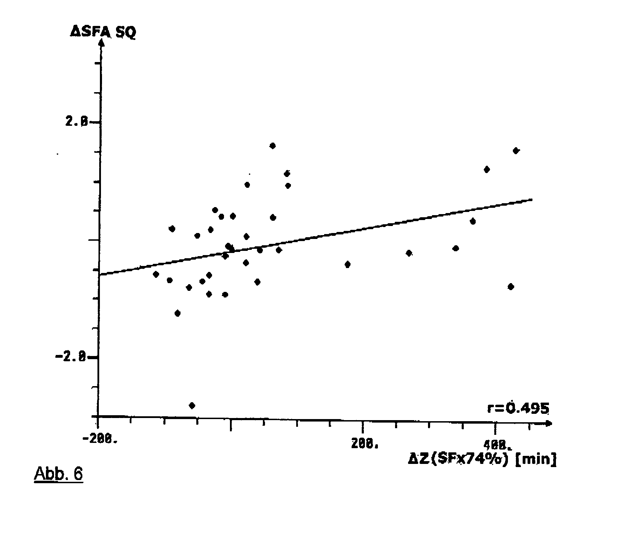

Abb. 6 zeigt die Korrelation zwischen dem Surrogatparameter für Schlaftiefe, der auf der Basis der quantitativen EEG Auswertung validiert wurde und dem subjektiven Gefühl, gut geschlafen zu haben, erfasst mittels Fragebogen. Gezeigt werden Änderungen der beiden Parameter unter Berücksichtigung aller Nächte in Gegenwart der verschiedenen Extraktmischungen.

In vivo Versuche an der Ratte zeigen Ergebnisse, die die Wirksamkeit einer Kombination von Orangenextrakt mit einem Extrakt der Baldrianwurzel und/oder Hopfenextrakt belegen.

Klinische Versuche an Patienten mit nachgewiesenen Schlafstörungen zeigen, daß die Kombination von Orangenextrakt mit Baldrianwurzelextrakt oder Hopfenextrakt beiden konventionellen Präparaten, d.h. Baidrianwurzetextrakt oder Hopfenextrakt, allein gegeben in statistisch signifikanter Weise überlegen ist. Die beste Wirkung war bei Gabe einer Dreierkonnbination bestehend aus Baidrianwurzetextrakt, Hopfenextrakt und Orangenextrakt zu verzeichnen.

Die Versuche wurden mit dem Orangenextrakt "Hesperidina Farma" der Firma Exquim S.A.; 08029 Barcelona, Spanien, durchgeführt, der ein Trockenextrakt in Form eines gelb-braunen Pulvers ist und 97% Hesperidin enthält.

Als Baldrianextrakt wurde ein Trockenextrakt "Valeriane extractum hydroaicoholicum siccum Ph. Eur. (91% nativ), DEV 3-6:1 in Ethanol 70%" eingesetzt. Das Pulver besteht aus 91% Extrakt und 9% Zusatzstoffen (8% Maltodextrin Ph. Eur. und 1 % colloidal anhydrous silica Ph. Eur.).

Als Hopfenextrakt wurde ein Extrakt "Hopfenextrakt getrocknet 4% Xanthohumol", 100% nativ, DEV 12,6-15,1 : 1 in Alkohol 5096 eingesetzt. Der Anteil an Xanthohumol und Isoxanthohumol beträgt mind. 4%.

Die Veränderungen der EEG-Frequenzen wurde nach Gabe von Salzlösung (Kontrolle bzw. Placebo) bzw. oral von Orangenextrakt in verschiedenen Kombinationen mit anderen Extrakte bestimmt.

Die Untersuchungen wurden analog der durch W. Dimpfel beschriebenen Methode (Dimpfel W: Preclinical data base of pharmaco-specific rat EEG fingerprints (Tele-Stereo-EEG). Eur J Med Res (2003) 8: 199-207) folgendermaßen durchgeführt: Sechs männlichen erwachsenen Fischer-344 Ratten (Tag-Nacht konvertiert) wurden im Alter von 6 Monaten 4 bipolar konzentrische Elektroden zusammen mit einem Mikrostecker auf einer gemeinsamen Basisplatte implantiert. Der Stecker diente der Aufnahme eines 4-Kanal-Senders zur telemetrischen Übertragung der aus frontalem Kortex, Hippocampus, Striatum und Formatio Reticularis abgeleiteten Feldpotentiale. Die Signale wurden auf einem Computer System (Software "EEG Anafyse", Betriebssystem OS Science, Laborrechner "LabTeam" der Firma MediSyst, Linden, DE) in Echtzeit einer Fast-Fourier-Transformation unterworfen und die Leistungsdichtespektren jeweils Ober 60 Minuten gemittelt. Die Unterteilung der Spektren in 6 verschiedene Frequenzbereiche erlaubte die Erfassung pharmako-spezifischer Veränderungen in Bezug auf die jeweils vor Applikation gemessenen Vorwerte innerhalb dieser Frequenzbänder.

Zum Applikationsprotokoll der Präparate: die Präparate wurden oral 45 Minuten nach Beginn der Messungen (Vorwert) appliziert Fünf Minuten später wurden die Messungen wieder gestartet, mindestens über die nächsten 5 Stunden kontinuierlich analysiert und in 60-minütigen Perioden zusammengefaßt. Die Testpräparate wurden in unterschiedlichen Kombinationen appliziert. Die experimentelle Serie wurde mit der Injektion von Salzlösung (Kontrolle), die zu keiner auffälligen Änderung führte, begonnen.

Der statistische Vergleich der Versuche zu den Ergebnissen, die nach Gabe von Salzlösung gemessen wurde, erfolgte nach Wilcoxon, Mann and Whitney auf der Basis der Veränderungen innerhalb der einzelnen Frequenzbänder in allen Hirnregionen als Variablen.

Die Verabreichung von Salzlösung.(Placebo) oder Orangenextrakt führte kaum zu Veränderungen der elektrischen Aktivität µV²/Ω) im Vergleich zu den Vorphasenwerten (Abb. 1). Bei den Untersuchungen der Veränderungen der EEG-Frequenzen der Ratte wurde aber überraschenderweise im Vergleich zur alleinigen Gabe von Baldrianextrakt oder Hopfenextrakt eine deutlich stärkere Zunahme der theta und beta1 Wellen nach Kombination mit Orangenextrakt gefunden, die bei einer Behandlung von Schlafstörungen nützlich ist, da die Zunahme dieser Frequenzen mit der Schlaftiefe korreliert (Abb. 1).

Die Verabreichung von Orangenextrakt in Kombination mit Baldrianwurzelextrakt oder Hopfenextrakt, führte zu stabilen Veränderungen der Leistungsdichte in allen Himgebieten, vor allem während der dritten bis fünften Stunde nach Applikation (siehe Abb. 1).

Die orale Verabreichung von Extraktkombinationen als Einzeldosis führte zu Veränderungen der elektrischen Himaktivität bei den Testtieren, die denen während des Schlafes ähnelten. Insbesondere ist dies überraschend, da die Präparate am Morgen, d.h. nach der Nachtruhe verabreicht wurden.

Die Abb. 2 zeigt die zeitabhängigen Veränderungen der theta EEG Frequenzen nach oraler Verabreichung verschiedener Dosierungen von Orangenextrakt, Baldrianwurzelextrakt und Hopfenextrakt allein sowie als Dreierkombination. In früheren Versuchen konnte gezeigt werden, daß vor allem die theta und beta1 Wellen des EEG die Schlaftiefe repräsentieren (Dimpfel W, Hofmann H-C Mehrdimensionale Dokumentation des Schlafes auf der Grundlage der Frequenzanalyse, In Mayer G (Hrgs. Jahrbuch Schlafmedizin Deutschland 1994: MMV Verlag, München). Obwohl der Orangenextrakt bei alleiniger Gabe keine Erhöhung der theta Frequenzen bewirkt, ist zu erkennen, daß die trotz hoher Dosis ebenfalls nur geringe Wirkung von Baidhanwurzeiextrakt oder Hopfenextrakt bei der Einzelapplikation durch die Kombination von Orangenextrakt, Baldrianwurzelextrakt und Hopfenextrakt massiv überboten wird und damit eine größere Schlaftiefe erreicht wird. Zudem ist in der Kombination nur die halbe Dosis beider klassischen Extrakte vorhanden, was auf eine Potenzierung durch den Orangenextrakt schließen läßt.

Ähnliches trifft für die für die Schlaftiefe wichtige beta1 Frequenz zu. Wie in Abb. 3 dargestellt übertrifft die Wirkung der Kombination die Wirkung beider Einzelextrakte deutlich, wobei Orangenextrakt allein keine Wirkung erkennen läßt.

Diese Wirkung konnte bei Schlaf-gestörten Patienten bestätigt werden. Nachdem der auf der Basis der theta und beta Frequenzen definierte spektrale Frequenzindex (Apparatus for determining sleep staging: US Pat. No. 6,157,857 Dec 05, 2000) an 12 Patienten in Gegenwart entweder von Placebo oder in Gegenwart der Dreiertcombination bzw. Zweierkombinationen mit Orangenextrakt während einer Nacht bestimmt worden war, ergab sich eine erheblich größere Schlaftiefe für die Dreierkombination im Vergleich zu beiden Zweierkombinationen (Es wurden immer dieselben Patienten im Abstand von mehreren Tagen untersucht). Das Ergebnis der Studie ist in Abb. 4 dokumentiert.

Betrachtet man darüber hinaus, wie viel Zeit die Patienten innerhalb einer jeweils bestimmten Schlaftiefe verbracht haben, ergibt sich eine klare Überlegenheit der Dreierkombination im Vergleich zu Placebo. Dies ist in Abb. 5 dokumentiert. Gleichzeitig ist ersichtlich, wie die Wirkung der Drelerkombination sich dem gesunden Schlaf nähert. Aus der Erkenntnis, das Orangenextrakt die für Schlaftiefe repräsentativen Parameter signifikant beeinflußt, muß geschlossen werden, daß der Zusatz von Orangenextrakt den Schlaf von Patienten deutlich bessert, was bislang unbekannt und auch nicht zu vermuten war.

Die Validität des anhand der EEG Aufzeichnung ausgewerteten und patentierten Surrogatparameters "Spektraler Frequenzindex" (SFx) wird auch durch die Korrelationsanalyse zwischen der Änderung des SFx und der anhand eines Fragebogens erfaßten subjektiven Qualität des Schlafes belegt. Die bereits anhand einer früheren Studie publizierte Korrelation (Dimpfel W, Hoffmann H-C, Schober F, Todorova A Validation of an EEG-derived spectral frequency index (SFx) for continuous monitoring of sleep depth in humans. Eur J Med Res 1998; 3: 453-460) konnte auch in dieser Studie wieder belegt werden, wie in Abb. 6 erkennbar ist. Daher muß auf Grund dieser Ergebnisse angenommen werden, daß eine Kombination pflanzlicher Schlafmittel mit Orangenextrakt zu einem hochpotenten Naturheilmittel zur Behandlung von Schlafstörungen mit geringem Nebenwirkungspotential führen wird.

Die polysomnographischen EEG,Daten wurden hauptsächlich mit dem spektralen Frequenzindex (SFx) ausgewertet, der Skala zwischen 0% bis 100% ein objektives Maß der Schlaftiefe liefert (Dimpfel W, Hoffmann H-C, Schober F, Todorova A: Validation of an EEG-derived spectral frequency index (Sf=x) for continuous monitoring of sleep depth in humans. Eur J Med Res 1998; 3: 453-460). Dieser neue Parameter, als spektraler Frequenzindex (SFx) bezeichnet, erfaßt die Schlaftiefe kontinuierlich und somit auch die gleitenden Übergänge der ca. 90 Minuten dauernden einzelnen Schlafphasen. Dieser Parameter skaliert zwischen 0 und 100%, wobei 0% auf der Basis keiner vorhandenen EEG Aktivität definiert ist, während 100% einen maximalen hyperwachen Zustand beschreibt, wie er nach Gabe von Stimulantien erreicht wird. Der Übergang vom Wachzustand in den Schlaf findet ab etwa 83% statt. Da unterhalb von 77% von sicherem Schlaf auszugehen ist, wurde der Parameter Schlafdauer auf der Basis der unterhalb dieser Schlaftiefe verbrachten Zeit definiert. Dadurch werden u.a. auch alle Aufwachvorgänge, die bekanntlich zu wenig erholsamen Schlaf führen, automatisch mit erfaßt und ausgewertet. Mit Hilfe dieses Parameters konnte eine Gruppe von Insomnikern von gesunden Schläfern (Auswertung von Daten einer früheren Studie) mit einer Irrtumswahrscheinlichkeit von p<0.005 unterschieden werden.

Das zu prüfende Hauptzielkriterium in der gegenwärtigen Studie war die Schlafdauer in Minuten auf der Basis des validierten Schlaftiefenindex SFx bei 74% Schlaftiefe. In dieser Studie wurde die gesamte Schlafdauer (S-Dauer) als einziger konfirmatorischer Zielparameter auf der Basis des validierten Schlaftiefenindex SFx verwendet (Dimpfel W, Suter A: Asleep improving effects of a single dose administration of a valerian/hops fluid extrakt. A double blind, randomized, placebo-controlled sleep-EEG study in a parallel design using the electrohypnogram, Eur J Med Res 2008; 13: 200-204). Dieser Zielparameter S-Dauer wird in Abhängigkeit vom SFx (t = Zeit) wie folgt definiert: Schlaf-Dauer: = Integral (von 0 bis 480 min) über die Zeit f(SFx(t)) dt, wobei f(SFx(t)) = min (1, (max (0, (80 - SFx(t))/3)) ist. (min = Minimum, max = Maximum). Die Schlaf-Dauer beschreibt die Zeitdauer des Schlafes von 22:00 Uhr - 6:00 Uhr morgens (8 h = 480 min). f(SFx(t) ist Null, wenn SFx >= 80%, und 1, wenn SFx <= 77%. Ist SFx = 79 %, so wird diese Minute als 1/3 Min gesetzt, ist SFx = 78%, so wird diese Minute als 2/3 Minute gewichtet. Dies entspricht einem "quasi" stetigen Übergang von SFx = 80% (f=0) zu SFx = 77% (f=1) mit einer Drittelgewichtung des Überganges. Dies beruht auf den Erkenntnissen, daß bei SFx = 80% der Schlaf übergangsweise eintritt und bei 77% der Schlaf sicher gegeben ist. Das Integral ergibt also die Gesamtzeit, in der der Patient einen SFx-Wert von "<80%" aufweist mit einer Drittelgewichtung im Übergangsbereich SFx = 80 - 77%, das insgesamt der Gesamtschlafzeit entspricht.

Die vorgenannten Beispiele dienen der Erläuterung der Erfindung, ohne daß die Erfindung jedoch auf die speziell beschriebenen Ausführungsformen beschränkt sein soll.

## Patentansprüche

1. Wirkstoffkombinationen, enthaltend
(i) Hesperidin, dessen pharmazeutisch annehmbare Salze und/oder dessen Aglykon Hesperetin;
und
(ii) mindestens einen weiteren Wirkstoff zur Behandlung von Schlafstörungen,

2. Wirkstoffkombinationen nach Anspruch 1, **dadurch gekennzeichnet, daß** sie Hesperidin in Form eines Pfianzenextrakts, bevorzugt eines Zitrusfruchtextrakts, insbesondere in Form von Orangenextrakt enthalten.

3. Wirkstoffkombinationen nach Anspruch 1 oder 2, **dadurch gekennzeichnet, daß** der mindestens eine weitere Wirkstoff ausgewählt ist aus pflanzlichen Wirkstoffen zur Behandlung von Schlafstörungen, Insbesondere aus Baldrian(wurzel) und/oder Hopfen(zapfen).

4. Wirkstoffkombinationen nach Anspruch 3, **dadurch gekennzeichnet, daß** der pflanzliche Wirkstoff in Form eines Pflanzenextrakts eingesetzt wird.

5. Schlafmittel oder Nahrungsergänzungsmittel umfassend eine Wirkstoffkombination, enthaltend Hesperidin, dessen pharmazeutisch annehmbare Salze und/oder dessen Aglykon Hesperetin und mindestens einen weiteren Wirkstoff zur Behandlung von Schlafstörungen.

6. Schlafmittel oder Nahrungsergänzungsmittel nach Anspruch 5, **dadurch gekennzeichnet, daß** sie Hesperidin in Form eines Pflanzenextrakts, bevorzugt eines Zitrusfruchtextrakts, insbesondere in Form von Orangenextrakt enthalten.

7. Schlafmittel oder Nahrungsergänzungsmittel nach Anspruch 5 oder 6, **dadurch gekennzeichnet, daß** der mindestens eine weitere Wirkstoff ausgewählt ist aus pflanzlichen Wirkstoffen zur Behandlung von Schlafstörungen, insbesondere aus. Baldrian(wurzel) und/oder Höpfen(zapfen), bevorzugt in Form eines Pflanzenextrakts.

8. Verwendung von Wirkstoffkombinationen, enthaltend Hesperidin, dessen pharmazeutisch annehmbare Salze und/oder dessen Aglykon Hesperetin und mindestens einen weiteren Wirkstoff zur Behandlung von Schlafstörungen.

9. Kombination von Orangenextrakt mit Baidrianwurzeiextrakt und/oder Hopfenextrakt zur Behandlung von Schlafstörungen.

10. Kombination nach Anspruch 9, **dadurch gekennzeichnet; daß** sie von 200 bis 800 mg Orangenextrakt, insbesondere von 400 bis 600 mg enthält.

11. Kombination nach Anspruch 9 oder 10, **dadurch gekennzeichnet, daß** sie von 50 bis 300 mg Hopfenextrakt, insbesondere von 100 bis 200 mg enthält.

12. Kombination nach einem der Ansprüche 9-11, **dadurch gekennzeichnet, daß** sie von 200 bis 800 mg Baldrianwurzelextrakt, insbesondere von 400 bis 600 mg enthält.

13. Schlafmittel oder Nahrungsergänzungsmittel nach einem der Ansprüche 5 - 7, **dadurch gekennzeichnet, daß** sie zusätzlich pharmazeutisch annehmbare Träger, Hilfs- und/oder Verdünnungsmittel enthalten.

14. Schlafmittel oder Nahrungsergänzungsmittel nach einem der Ansprüche 5 - 7 oder 13, **dadurch gekennzeichnet, daß** sie zur oralen Verabreichung hergerichtet sind.
